# EUROPÄISCHE PATENTSCHRIFT

(11) **EP 3 287 833 B1**
(45) Veröffentlichungstag und Bekanntmachung des Hinweises auf die Patenterteilung: **06.12.2023**
(21) Anmeldenummer: 17186426.7
(22) Anmeldetag: 16.08.2017
(51) Int. Cl.: G02B 27/00, A61M 1/14

(54) **VORRICHTUNG ZUR EXTRAKORPORALEN BLUTBEHANDLUNG MIT MONITOREINRICHTUNG**
DEVICE FOR EXTRACORPOREAL BLOOD TREATMENT WITH MONITOR
DISPOSITIF DE TRAITEMENT EXTRACORPOREL DU SANG DOTÉ D'UN DISPOSITIF DE SURVEILLANCE

(30) Priorität: 17.08.2016 DE 102016115270
(43) Veröffentlichungstag der Anmeldung: 28.02.2018
(73) Patentinhaber: B. Braun Avitum AG, 34212 Melsungen (DE)
(72) Erfinder: SCHÄFER, Oliver, 36286 Neuenstein (DE); SCHLEICHER, Christian, 36160 Dipperz (DE)
(74) Vertreter: Winter, Brandl - Partnerschaft mbB

(56) Entgegenhaltungen:
- WO-A2-2008/100983
- GB-A- 2 428 153
- US-A- 5 528 319
- US-A1- 2004 218 245
- US-A1- 2005 111 100
- US-A1- 2007 112 603
- US-A1- 2009 222 119
- US-A1- 2010 091 226
- US-A1- 2011 018 860
- US-A1- 2012 154 264
- US-B2- 6 379 788

## Beschreibung

Die Erfindung betrifft eine Vorrichtung zur extrakorporalen Blutbehandlung wie beispielsweise eine Dialysemaschine, die eine Monitoreinrichtung in Form beispielsweise eines Bildschirms mit einer Anzeigeeinrichtung beinhaltet.

Im Stand der Technik sind bei Maschinen zur extrakorporalen Blutbehandlung, beispielsweise Dialysemaschinen, feststehende Monitoreinrichtungen bekannt. Solche feststehende oder zumindest nicht neigbare Monitore der bekannten Art sind bislang dahingehend nachteilig, dass unter ungünstigen Bedingungen, beispielsweise unter ungünstigem Lichteinfall auf eine Anzeigeeinrichtung des feststehenden Monitors oder ungünstiger Position des Geräts, eine Benutzeroberfläche nicht oder nur schwer erkennbar ist, und die Ablesbarkeit von auf der Anzeigeeinrichtung dargestellter Information und/oder einer Benutzeroberfläche beeinträchtigt und die Information nicht genau und/oder nur schwer zu erkennen ist.

Da ein einfaches Drehen des Monitors hin zu günstigerem Lichteinfall bei einem feststehenden Monitor nicht möglich und die Drehbarkeit des Geräts in situ in der Regel ebenfalls eingeschränkt ist, kann eine die Ablesbarkeit gegebenenfalls verbessernde Drehung des gesamten Geräts weiter nachteilig dazu führen, dass beispielsweise Datenschutzbestimmungen entgegen Information, beispielsweise vertrauliche Patientendaten, Personen zugänglich wird, für die sie nicht bestimmt ist.

Um dem entgegenzuwirken, sollten daher bekannte Geräte, wie etwa Dialysemaschinen, separat und von nicht autorisierten Personen nicht einsehbar ausgerichtet werden. Zwar können bei vernetzten Maschinen beispielsweise Behandlungsparameter und/oder Patientendaten in etwa einem Schwesternzimmer mittels einer Netzwerkschnittstelle darstellbar und betrachtbar sein. Bislang ist es jedoch nicht möglich, direkt an einer Maschine Patientendaten und/oder Parameter einer benachbarten Maschine einzusehen, ohne Information in unerwünschter Weise preiszugeben oder die Einhaltung von Datenschutzbestimmungen zu gefährden.

Gattungsgemäßer Stand der Technik ist aus den Dokumenten US 2009 / 222 119 A1, US 2012/154 264 A1 sowie den Dokumenten US 2010 / 091226 A1, US 2007 / 112 603 A1, GB 2 428 153 A und US 2005 111 100 A1 bekannt. Als weiterer Stand der Technik sind WO 2008/100893 A2, US 2011/018860 A1, US 6 379 788 B2, US 5 528 319 A und US 2004/218245 A1 zu nennen.

Der Erfindung liegt daher als eine Aufgabe zugrunde, die vorstehenden Nachteile zu überwinden und bei einer Vorrichtung zur extrakorporalen Blutbehandlung, insbesondere einer Dialysemaschine, mit einer feststehenden Monitoreinrichtung die Ablesbarkeit von Anzeigeinhalten auf einer Anzeigeeinrichtung derselben bei gleichzeitig erhöhtem Datenschutz praxistauglich und anwenderfreundlich zu verbessern.

Diese Aufgabe wird erfindungsgemäß gelöst durch ein medizintechnisches Gerät mit den Merkmalen des Anspruchs 1. Vorteilhafte Weiterbildungen der Erfindung sind Gegenstand der beigefügten Unteransprüche. Die Erfindung ist im beigefügten Anspruchssatz beschrieben.

Der Erfindung liegt als eine allgemeine Idee zugrunde, bei einem feststehenden und/oder drehbaren Monitor, welcher nicht neigbar ist, die Erkennbarkeit einer Benutzeroberfläche zu verbessern, d.h. an beispielsweise einem Gerät zur extrakorporalen Blutbehandlung, wie einer Dialysemaschine und dergleichen, die eine feststehende und/oder integrierte bzw. nicht ausrichtbare Monitoreinrichtung (Monitor) mit einer Anzeigeeinrichtung aufweist, die Lesbarkeit von auf der Anzeigeeinrichtung dargestellter Information durch gezielte Maßnahmen oder zusammenwirkende Kombination gezielter Maßnahmen zu beeinflussen.

Diese Vorteile werden mit einer Anordnung erzielt, die einen teilbaren Monitorinhalt bereitstellt. Teilbar im hier vorliegenden Sinne bezieht sich auf die gleichzeitige Darstellung zumindest zweier verschiedener Bildschirm/Anzeige/Monitorinhalte, von welchen aus einer vorbestimmten Blickrichtung und/oder Entfernung einer der Bildschirm/Anzeige/Monitorinhalte und aus einer anderen vorbestimmten Blickrichtung und/oder Entfernung ein anderer der Bildschirm/Anzeige/Monitorinhalte sichtbar sind. Ferner umfassen die gezielten Maßnahmen eine blickwinkelabhängige Informationsdarstellung über gesteuerte Flüssigkristalle, eine Reflexminderung bzw. Entspiegelung und ggf. Polarisation einer Anzeigeoberfläche durch beispielsweise Schicht- oder Folienauftrag auf oder -integration in der Anzeigeoberfläche oder Anordnung einer Schicht oder Folie vor der Anzeigeoberfläche, und eine zumindest teilweise bzw. abschnittsweise Verspiegelung der Anzeigeoberfläche, wodurch auch bei ungünstigem Lichteinfall auf die Anzeigeoberfläche die Notwendigkeit eines Drehens des Geräts verringert wird, und durch gezielte bzw. im Hinblick auf einen Betrachtungswinkel eines Anwenders gerichtete Darstellung von Information der Datenschutz erhöht werden kann und sich gleichzeitig die Information auch unter ungünstigen Bedingungen gut lesen bzw. darstellen lässt.

Bei einer blickwinkelabhängigen Informationsdarstellung über gesteuerte Flüssigkristalle können zum Beispiel bei orthogonalem Blickwinkel mit einer Toleranz von beispielsweise +/- 10° spezielle Therapieparameter (z.B. kT/V, ein HK-Wert und dergleichen) und Einstellungsmöglichkeiten angezeigt werden, während bei einer Betrachtung unter einem Winkel größer als beispielsweise 15° (20°, 25°, ...) bis hin zu beispielsweise 30° zu der Orthogonalen allgemeine Informationen wie beispielsweise eine Behandlungsdauer, eine Uhrzeit und dergleichen dargestellt werden können. Eine derartige Darstellung ist sowohl einseitig als auch beidseitig konfigurierbar und anwenderseitig über Maschineneinstellungen änderbar. In diesem Fall kann die Bilddarstellung außerhalb der definierten Betrachtungswinkel als gespiegelte bzw. verspiegelte oder spiegelnde Oberfläche erscheinen. Technisch umsetzbar ist dies zum Beispiel durch Anlegen einer elektrischen Spannung an Flüssigkristalle, welche die Polarisationsrichtung des Lichts beeinflussen und dadurch unterschiedliche Anzeigen bzw. Einblickwinkel ermöglichen.

Alternativ oder zusätzlich sind Polarisationsebenen in Kombination mit einer Prismenstruktur (letztere zur Erhöhung der Polarisation) auf einen festen Betrachtungswinkel einstellbar, so dass Streulicht verbessert in unterschiedliche Richtungen abgeleitet wird. Auf diese Weise kann eine Blendung des Anwenders verringert werden. Um den Grad der Polarisation zu erhöhen, sind mehrere Einzelpolarisationen hintereinander positionierbar.

Eine verbesserte Ableitung von Streulicht und Verringerung der Blendung des Anwenders ist ferner durch Entspiegelung mittels einer Beschichtung der Display- oder Anzeigeoberfläche, beispielsweise in Form einer Antireflexbeschichtung (AR-Beschichtung), mittels Prismen, Linsen, einer rauen Oberfläche und dergleichen, erzielbar.

Der Reflexionsgrad der AR-beschichteten Fläche kann durch eine destruktive Interferenz der reflektierten Strahlen erreicht bzw. beeinflusst werden. Zugunsten einer diesbezüglichen Reflexminderung kann beispielsweise eine nach dem Prinzip destruktiver Interferenz arbeitende, nicht absorbierende Schicht (Anti-Reflex-Schicht) eines Stoffes oder einer Verbindung von Stoffen mit vorbestimmter Brechzahl und Dicke auf ein Substrat aufgedampft sein. Grenzt diese Aufdampfschicht mit einer vorbestimmten Brechzahl an Luft, können sich Wellenzüge des an der Schicht und der darunter liegenden Oberfläche reflektierten Lichts durch Interferenz auslöschen, sofern eine Phasenbedingung und eine Amplitudenbedingung als Interferenzbedingungen erfüllt sind. Falls sich durch eine einzelne Schicht mit einheitlicher Brechzahl die vorgenannten Interferenzbedingungen nur für eine Wellenlänge exakt erfüllen lassen, können mehrere Schichten mit unterschiedlichen Brechzahlen als insgesamt breitbandige Anti-Reflex-Schicht aufgebracht sein. Ferner ist es möglich, durch Laminieren beispielsweise eines LCD- oder LED-Panels direkt, d.h. ohne Luftspalt, an eine Anzeigeoberfläche Fresnel-Reflexionen zu unterdrücken, und/oder durch Plasmaätzen eine Entspiegelung zu erzielen.

Eine Entspiegelung kann somit Transmission und Kontrast verbessern und eine gute Sichtbarkeit des Bildschirm- oder Anzeigeinhalts aus mehreren, im Idealfall allen Richtungen ermöglichen. Ein Anwender kann auch bei ungünstigem Lichteinfall direkt Parameteränderungen vor und während einer Behandlung vornehmen, ohne zunächst den Monitor aus einem Blickwinkel mit Blendung bzw. Blendwinkel drehen zu müssen. Durch eine gezielte Verspiegelung, Polarisation und/ oder Kontraständerung kann erreicht werden, dass vertrauliche Inhalte über beispielsweise einen Patienten nur aus einer bestimmten Position ablesbar sind. Besucher oder auch andere Patienten können in diesem Fall diese vertraulichen Daten nicht mehr ohne weiteres einsehen, wodurch ein erhöhter und verbesserter Datenschutz bereitgestellt wird. Darüber hinaus ermöglicht diese Konfiguration, in beispielsweise einem vernetzten Dialysezentrum Parametereinstellungen einer anderen Maschine anzuzeigen, da diese nur von einer befugten Person (z.B. Schwester) abgelesen werden kann. Somit können Parameter unterschiedlicher Patienten ohne großen Aufwand verglichen werden, ohne dass diese Information von Dritten einsehbar ist.

Die Vorteile der Erfindung werden mit einer Anordnung erzielt, die einen teilbaren Monitorinhalt bereitstellt. Teilbar im hier vorliegenden Sinne bezieht sich auf die gleichzeitige Darstellung zumindest zweier verschiedener Bildschirm/Anzeige/Monitorinhalte, von welchen aus einem vorbestimmten Betrachtungswinkel bzw. Betrachtungswinkelbereich und/oder aus einer vorbestimmten Entfernung bzw. einem vorbestimmten Entfernungswinkelbereich nur einer der Bildschirm/Anzeige/Monitorinhalte einsehbar ist und aus einem anderen vorbestimmten Betrachtungswinkel bzw. Betrachtungswinkelbereich und/oder aus einer anderen vorbestimmten Entfernung bzw. einem vorbestimmten Entfernungswinkelbereich der Blickrichtung nur ein anderer der Bildschirm/Anzeige/Monitorinhalte einsehbar ist. Als Beispiel kann etwa eine Alarmanzeige oder Alarmmeldung genannt werden, welche nur bei größerer Entfernung zu dem Monitor bzw. Bildschirm sichtbar ist, während nahe vor dem Monitor ein anderer Anzeigeinhalt sichtbar ist oder bei Annäherung an den Monitor sichtbar wird, welcher beispielsweise eine Menüführung und Detailinformation beinhalten kann. Es werden, wie vorstehend erwähnt, jedenfalls beide Anzeigeinhalte dargestellt, so dass der Anzeigeinhalt für einen einzelnen, seine Position verändernden Anwender wechselt, oder von zwei Anwendern an unterschiedlichen Positionen jeweils unterschiedliche Information von ein und demselben Bildschirm ablesbar sind. Für einen ersten Anwender, der sich beispielsweise mehrere Meter von der Maschine entfernt befinden kann, ist die vorgenannte Alarmmeldung oder Alarmanzeige sichtbar, und für einen zweiten Anwender, der sich beispielsweise unmittelbar vor der Maschine befinden kann, sind Detailinformationen und eine Menüführung sichtbar.

Im Einzelnen werden die vorstehenden Vorteile realisiert und die Aufgabe gelöst durch eine Vorrichtung zur extrakorporalen Blutbehandlung und bevorzugt eine Dialysemaschine, mit einem Gehäuse zur Aufnahme betrieblicher Komponenten für die extrakorporale Blutbehandlung, mit einem Korpus und einer bezüglich des Korpus feststehenden bzw. nicht ausrichtbaren oder zumindest nicht neigbaren Monitoreinrichtung mit einer Anzeigeeinrichtung, wobei die Monitoreinrichtung eine Anzeigeinhaltsdarstellungs-Beeinflussungseinrichtung aufweist, die dazu angeordnet ist, die Einsehbarkeit von auf der Anzeigeeinrichtung dargestellter Information zu beeinflussen.

Die Anzeigeeinrichtung ist dazu angeordnet, zumindest zwei unterschiedliche Anzeigeinhalte gleichzeitig anzuzeigen, und die Anzeigeinhaltsdarstellungs-Beeinflussungseinrichtung ist dazu angeordnet, die Sichtbarkeit der auf der Anzeigeeinrichtung gleichzeitig dargestellten zumindest zwei Anzeigeinhalte derart zu beeinflussen, dass aus einer ersten Blinkrichtung und/oder Entfernung eines ersten Anwenders auf/zu die/der Anzeigeeinrichtung einer der zumindest zwei Anzeigeinhalte und aus einer zweiten Blinkrichtung und/oder Entfernung eines zweiten Anwenders auf/zu die/der Anzeigeeinrichtung ein anderer der zumindest zwei Anzeigeinhalte sichtbar sind.

Bevorzugt ist die Anzeigeinhaltsdarstellungs-Beeinflussungseinrichtung ein in die Anzeigeeinrichtung integriertes Polarisationsmittel oder Kontraständerungsmittel, das dazu angeordnet ist, dargestellten Anzeigeinhalt nur aus einer vorbestimmten Position oder Blickrichtung eines Anwenders lesbar zu machen.

Bevorzugt ist die Anzeigeeinrichtung als eine Dual-View-Anzeigeeinrichtung konfiguriert. Eine Dual-View-Konfiguration ermöglicht vorteilhaft die Darstellung unterschiedlicher Bildinhalte für mehrere Personen, die aus unterschiedlichen Perspektiven, Richtungen oder Blickwinkeln auf die Anzeigeeinrichtung blicken.

Bevorzugt ist die Anzeigeeinrichtung als eine Split-Screen-Anzeigeeinrichtung konfiguriert. Vorteilhaft ermöglich die Split-Screen-Konfiguration bei entsprechender Aufteilung der Fläche der Anzeigeeinrichtung die getrennte Darstellung verschiedener Bildinhalte, beispielsweise auch solcher, die über ein Netzwerk von einer entfernten Quelle, beispielsweise einem Arztplatz, einem Dienstleister zu Wartungs- oder Hilfestellungszwecken, oder einem anderen medizinischen Gerät, bereitgestellt werden. In Kombination mit der Dual-View-Konfiguration ist es weiter vorteilhaft möglich, derartige Bildinhalte für Unberechtigte uneinsehbar zu halten.

Die Anzeigeinhaltsdarstellungs-Änderungseinrichtung ist ein zur Änderung seiner Lichttransmissionseigenschaften elektronisch ansteuerbares Filter, das als eine Parallaxenbarriere konfiguriert ist. Vorteilhaft sind auf diese Weise beliebige Betrachtungsbereiche unterstützbar und/oder für einen oder mehrere Betrachter optimierbar. Vorteilhaft verwendbar sind beispielsweise mittels nach dem Parallaxenbarrierenprinzip arbeitende schaltbare und dadurch lichtlenkende Flüssigkristalle, welche als zweite Flüssigkristallschicht vor oder hinter einem Panel in den Aufbau einer Anzeigeeinrichtung integriert sind und sich unabhängig von einer bildgebenden Anzeigeeinrichtung (TFT- oder LED-Panel) direkt ansteuern lassen. Eine Winkeldifferenz zwischen Bildachsen kann beispielsweise 40° betragen, wobei in diesem Fall Betrachter ab einem Abstand von beispielsweise 50 cm von der linken und rechten Seite aus unterschiedliche Bilder sehen können.

Zusätzlich kann die Anzeigeinhaltsdarstellungs-Beeinflussungseinrichtung eine vorgeschaltete Schicht, Scheibe oder Folie, die die Darstellung entspiegelt oder verspiegelt, und/oder eine die Sichtbarkeit des Anzeigeinhalts winkel- und/oder richtungsabhängig verändernde Teilvorrichtung bzw. Komponente der Anzeigeeinrichtung aufweisen.

Vorteile ergeben sich insbesondere bei einer Dialysemaschine, bei welcher erhöhter Beobachtungsaufwand und erhöhte Datenschutzanforderungen bestehen können und angezeigte Parameter und/oder Werte nicht jedermann zugänglich sein sollen.

Die Anzeigeinhaltsdarstellungs-Beeinflussungseinrichtung beinhaltet ein auf einer Anzeigeoberfläche der Anzeigeeinrichtung angeordnetes Entspiegelungsmittel, das dazu angeordnet ist, zumindest einen Teilbereich der Anzeigeoberfläche zu entspiegeln. Das Entspiegelungsmittel kann ein Reflexminderungsmittel sein, das dazu angeordnet ist, Reflexionen zu verringern. Vorteilhaft kann dadurch auch bei ungünstigem Lichteinfall auf die Anzeigeoberfläche die Notwendigkeit eines Drehens des Geräts verringert werden, und lässt sich gleichzeitig die Information auch unter ungünstigen Bedingungen gut lesen bzw. darstellen. Eine Entspiegelung kann somit Transmission und Kontrast verbessern und eine gute Sichtbarkeit des Bildschirm- oder Anzeigeinhalts aus mehreren, im Idealfall allen Richtungen ermöglichen.

Bevorzugt ist das Entspiegelungsmittel eine in dem zumindest einen Teilbereich auf oder vor der Anzeigeoberfläche angeordnete Folie oder Beschichtung, oder eine Folie, eine Scheibe, ein Kontraständerungsmittel und/oder eine LED-Anordnung, die vor der Anzeigeoberfläche der Anzeigeeinrichtung angeordnet ist/sind.

Die Anzeigeinhaltsdarstellungs-Beeinflussungseinrichtung beinhaltet ein auf einer Anzeigeoberfläche der Anzeigeeinrichtung angeordnetes Verspiegelungsmittel, das dazu angeordnet ist, zumindest einen Teilbereich der Anzeigeoberfläche gezielt zu verspiegeln. Vorteilhaft kann durch gezielte bzw. im Hinblick auf einen Betrachtungswinkel eines Anwenders gerichtete Darstellung von Information der Datenschutz erhöht werden.

Die Erfindung wird nachstehend anhand bevorzugter Ausführungsbeispiele unter Bezugnahme auf die beigefügte Zeichnung näher beschrieben. Es zeigen:
Fig. 1 in einer vereinfachten Aufsicht ein medizinisches Gerät / eine Dialysemaschine mit einer Teil einer Monitoreinrichtung bildenden Anzeigeeinrichtung mit Blickwinkelsteuerung, bei welcher unterschiedliche Anzeigeinhalte aus unterschiedlichen Richtungen einsehbar sind, gemäß einem ersten Ausführungsbeispiel;
Fig. 2 in einer vereinfachten Aufsicht ein medizinisches Gerät mit einem Teil einer Monitoreinrichtung bildenden Anzeigeeinrichtung mit Blickwinkelsteuerung, bei welcher unterschiedliche Anzeigeinhalte aus unterschiedlichen Entfernungen einsehbar sind, gemäß einem zweiten Ausführungsbeispiel; und
Fig. 3 eine schematische, ausschnittweise Prinzipdarstellung der Anzeigeeinrichtung mit Blickwinkelsteuerung und optional entspiegelndem und/oder verspiegelndem Schichtauftrag gemäß dem ersten und dem zweiten Ausführungsbeispiel.

In der nachfolgenden Figurenbeschreibung werden in den einzelnen Figuren gleiche oder gleichwirkende Elemente und/oder Komponenten gleich und/oder mit denselben Bezugszeichen bezeichnet und zweckmäßig nicht redundant beschrieben. In Fällen, in welchen ein nachfolgendes Ausführungsbeispiel funktionell zumindest einem vorangehenden entspricht, d.h. entsprechende Funktionen, Anordnungen und/oder Verfahrens- oder Betriebsabläufe gleichermaßen umfasst sind, wird zweckmäßig nur auf Unterschiede eingegangen.

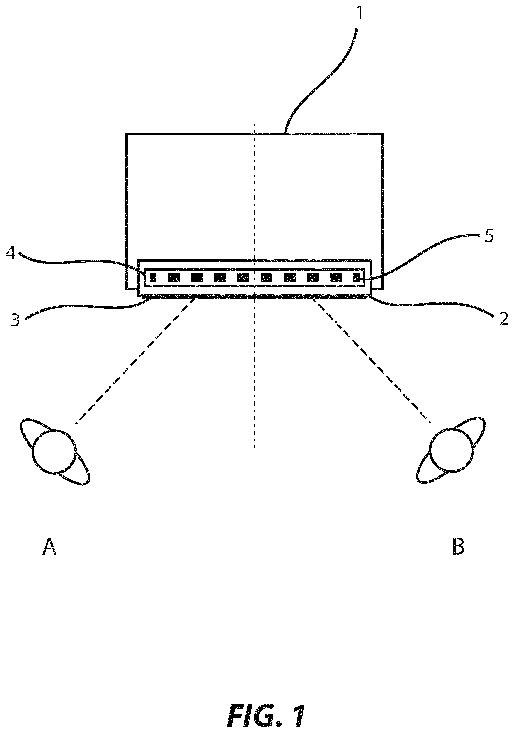

Fig. 1 zeigt in einer vereinfachten Aufsicht ein medizinisches Gerät / eine Vorrichtung zur extrakorporalen Blutbehandlung 1 mit einer Monitoreinrichtung 2, bei welcher unterschiedliche Anzeigeinhalte aus unterschiedlichen Richtungen einsehbar sind, gemäß einem ersten Ausführungsbeispiel. Nachstehend wird das erste Ausführungsbeispiel anhand zweier unterschiedlicher Anzeigeinhalte und entsprechend zweier unterschiedlicher Richtungen A, B beschrieben. Eine Beschränkung hierauf besteht jedoch nicht.

Bei dem medizintechnischen Gerät 1, insbesondere einer Dialysemaschine, ist eine Anzeigeeinheit oder Anzeigeeinrichtung der Monitoreinrichtung 2 nach einem so genannten Split-Screen- und/oder Dual-View-Prinzip konfiguriert bzw. aufgebaut und arbeitet dementsprechend.

Es wird angemerkt, dass im Fall der Dialysemaschine an dem Gehäuse bzw. dem Korpus eine Anzahl von Komponenten zur Durchführung einer Blutbehandlung in an sich bekannter Weise funktionsfähig zusammenwirkend angeordnet sind, wie beispielsweise eine Steuerelektronik, zumindest Teile der Hydraulik, Pumpen, Heizeinrichtungen und ggf. Tanks oder Beutel für ausgewählte Betriebsstoffe, welche sämtlich aus dem Stand der Technik hinlänglich bekannt sind und daher nicht weiter dargestellt oder beschrieben werden.

Die Monitoreinrichtung 2 weist eine Anzeigeeinrichtung 4 auf, in welcher noch näher zu beschreibende Blickwinkelsteuermittel 5 integriert sind, und nutzt einen einzelnen (beispielsweise Flüssigkristall-, TFT- oder LED-) Anzeigeschirm (Panel) zum gleichzeitigen Darstellen von zumindest zwei unterschiedlichen Stand- und/oder Bewegtbildern, die jeweils aus unterschiedlichen Betrachtungsrichtungen und/oder Entfernungen (Betrachtungsperspektive) isoliert sichtbar sind. In anderen Worten zeigt sich für einen Betrachter aus unterschiedlichen Perspektiven ein jeweils vollständig anderes Bild auf ein und demselben Bildschirm. Die Blickwinkelsteuermittel 5 sind eine Lichtrichtung ändernde Mittel, beispielsweise ein elektronisch ansteuerbares Lichtrichtungsänderungsfilter oder durch Anlegen einer elektrischen Spannung in ihrer Ausrichtung steuerbare Flüssigkristalle, und bilden eine Anzeigeinhaltsdarstellungs-Beeinflussungseinrichtung aus.

Auf der Oberfläche der Anzeigeeinrichtung 4 ist eine für die Oberfläche zumindest teilweise reflexmindernde bzw. entspiegelnde und verspiegelnde Beschichtung oder Schicht 3 aufgebracht. Alternativ und/oder gleichzeitig kann eine reflexmindernde bzw. entspiegelnde und verspiegelnde separate Komponente in Form beispielsweise eines platten- oder scheibenförmigen Elements vor der Oberfläche bereitgestellt sein.

Eine reflexmindernde Schicht kann beispielsweise als eine nach dem Prinzip destruktiver Interferenz arbeitende, nicht absorbierende Schicht (Anti-Reflex-Schicht bzw. AR-Schicht) eines Stoffes oder einer Verbindung von Stoffen mit vorbestimmter Brechzahl und Dicke auf ein Substrat aufgedampft sein. Grenzt diese Aufdampfschicht mit einer vorbestimmten Brechzahl an Luft, können sich Wellenzüge des an der Schicht und der darunter liegenden Oberfläche reflektierten Lichts durch Interferenz auslöschen, sofern eine Phasenbedingung und eine Amplitudenbedingung als Interferenzbedingungen erfüllt sind. Falls sich durch eine einzelne Schicht mit einheitlicher Brechzahl die vorgenannten Interferenzbedingungen nur für eine Wellenlänge exakt erfüllen lassen, können mehrere Schichten mit unterschiedlichen Brechzahlen als insgesamt breitbandige Anti-Reflex-Schicht aufgebracht sein.

Ferner ist es möglich, durch Laminieren beispielsweise eines LCD- oder LED-Panels direkt, d.h. ohne Luftspalt, an eine Anzeigeoberfläche Fresnel-Reflexionen zu unterdrücken, und/oder durch Plasmaätzen eine Entspiegelung zu erzielen.

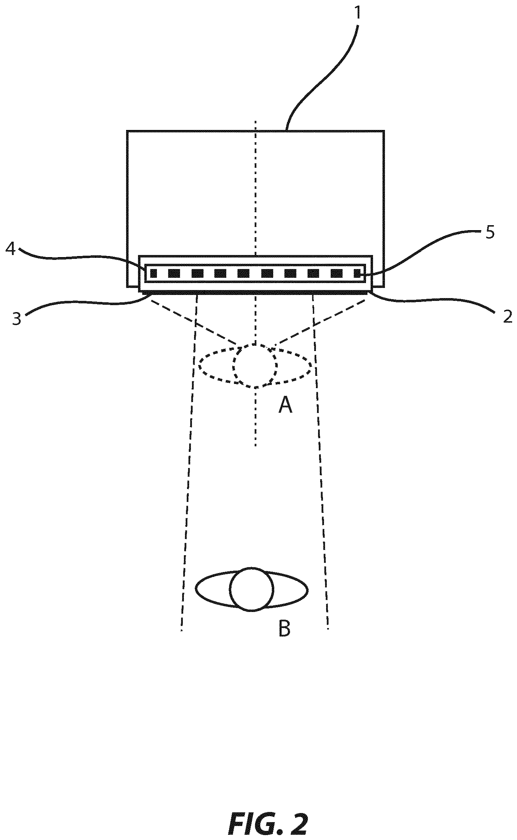

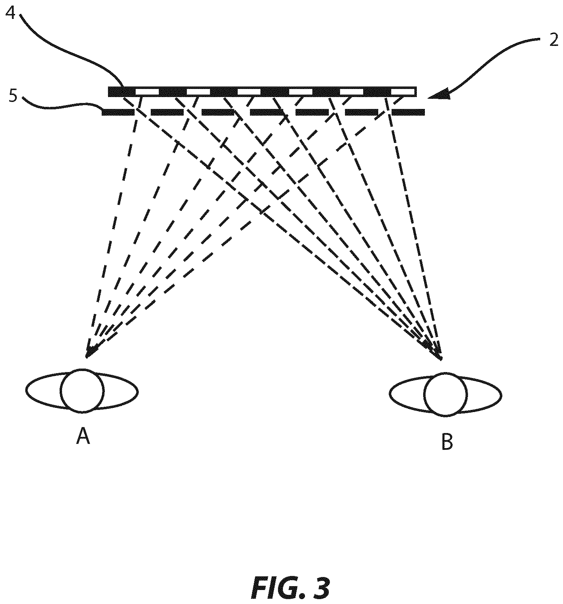

Fig. 2 zeigt in einer vereinfachten Aufsicht ein medizinisches Gerät mit einer Monitoreinrichtung 2, bei welcher unterschiedliche Anzeigeinhalte aus unterschiedlichen Entfernungen einsehbar sind, gemäß einem zweiten Ausführungsbeispiel. Die grundlegende Anordnung und Funktionsweise der Monitoreinrichtung 2 gemäß Fig. 2 entspricht hierbei derjenigen der Fig. 1. Eine beispielhafte Funktionsweise der Monitoreinrichtung 2 gemäß Fig. 1 und 2 wird nachstehend unter Bezugnahme auf Fig. 3 beschrieben.

Fig. 3 zeigt eine schematische, ausschnittweise Prinzipdarstellung einer Teil der Monitoreinrichtung 2 bildenden Anzeigeeinrichtung 4 mit Blickwinkelsteuerung und entspiegelndem und verspiegelndem Schichtauftrag, und stellt insoweit das Prinzip einer Parallaxenbarriere dar, nach dem ein Bildschirm oder Monitor zwar zwei unterschiedliche Bilder darstellt, wie in der Figur durch eine Folge von abwechselnd schwarz und weiß gefüllten Rechtecken auf einer Linie angedeutet (erstes Bild, schwarz gefüllte Rechtecke, und zweites Bild, weiß gefüllte Rechtecke), je nach Standort einer Person A oder B diese jedoch nur eines der Bilder sieht.

Die hierin beschriebene Anzeigeeinrichtung 4 mit Blickwinkelsteuerung kann auch nach einem Dual-View-Prinzip und/oder einem Bildaufteilungsprinzip (Split Screen) konfiguriert sein, das es erlaubt, zwei verschiedene Darstellungen von Bildinhalten für zwei aus verschiedenen Entfernungen und Richtungen betrachtende Betrachter zu erzeugen.

Beispielsweise sind hierzu so genannte Dual-Stacked- oder Doppelstapel-Flüssigkristallanzeigen bzw. -LCDs einsetzbar, bei welchen beliebige Betrachtungsbereiche unterstützbar und/oder für einen oder mehrere Betrachter optimierbar sind, und werden mittels nach dem Parallaxenbarrierenprinzip arbeitenden schaltbaren Flüssigkristallen, welche als Flüssigkristallschicht in den Aufbau einer Anzeigeeinrichtung (Panel) integriert sind und sich unabhängig von einer bildgebenden Anzeigeeinrichtung (beispielsweise eines LCD-, TFT- oder LED-Panels) direkt ansteuern lassen, Molekülstränge der Flüssigkristalle durch elektrische Signale zu einer Streifenmaske ausgerichtet, welche das Licht (Hintergrundlicht bzw. Backlight) nach links und rechts steuert, so dass das Licht gezielt in unterschiedliche Richtungen gelenkt werden kann.

In anderen Worten sind in den hierin beschriebenen Ausführungsbeispielen zumindest zwei Bilder gleichzeitig darstellbar, wobei mittels der speziellen LCD-Schicht, die vor oder hinter dem eigentlichen LCD-, TFT- oder LED-Panel angeordnet ist und als Filter arbeitet, das Licht einzelner Pixel in die unterschiedlichen Richtungen, beispielsweise in eine erste Richtung A und in eine zweite Richtung B, vor dem Bildschirm gelenkt wird und in die Augen jeder einer sich in der ersten Richtung A oder der zweiten Richtung B (vgl. Fig. 3) befindenden Person (Betrachter/Anwender) ein anderes Bild fällt.

Auf der Anzeigeeinrichtung 4 erscheinen die beiden Bilder daher in jeweils unterschiedlichen, dünnen Streifen, abwechselnd für ein linkes und ein rechtes Bild. Aufgrund dieser Streifenmaske sind Pixel für das linke Bild nur von links zu sehen, während die Maske nach rechts nur den Blick auf die Bildanteile des rechten Bilds freigibt. Dadurch ist es möglich, blickwinkel- oder richtungsabhängig zwei völlig verschiedene Inhalte auf demselben Bildschirm darzustellen. In diesem Fall sehen Betrachter, die aus verschiedenen Blickwinkeln, d.h. Perspektiven (vgl. Fig. 1, Fig. 3) oder Entfernungen (vgl. Fig. 2), auf die Monitoreinrichtung 2 blicken, jeweils unterschiedliche Bildinhalte.

Eine Winkeldifferenz zwischen den beiden Bildachsen kann dabei beispielsweise 40° betragen, wobei in diesem Fall Betrachter ab einem Abstand von beispielsweise 50 cm von der linken und rechten Seite aus unterschiedliche Bilder sehen können. Eine Beschränkung auf diese beispielsweisen Werte besteht jedoch nicht.

Wird derselbe visuelle Inhalt für beide Seiten zugeführt, kann die Anzeigeeinrichtung auch als normale Anzeigeeinrichtung genutzt werden.

Wie vorstehend erwähnt, kann eine Blickwinkel- bzw. Betrachtungswinkelumschaltung der in den Aufbau des TFT-Panels integrierten Schicht schaltbarer Flüssigkristalle durch elektronisches Ansteuern der Stränge der schaltbaren Flüssigkristallmoleküle (durch eine elektrische Spannung) und dadurch vorbestimmtes Ausrichten derselben, um den Strahlengang des Hintergrundlichts zu steuern, erfolgen. Die Doppelbrechung des Flüssigkristallmaterials kann dadurch so verändert werden, dass Licht nur noch nach vorn austreten kann und bei einem Einblick von oben oder von der Seite das Sichtfeld dunkel bleibt. Fällt die Spannung wieder ab, nehmen die Molekülstränge wieder einen optisch isotropen Zustand ein und lassen das Licht der Hintergrundbeleuchtung in jede Richtung passieren.

Wie vorstehend beschrieben wurde, kann durch geeignete Schaltzyklen und geeignete Umschaltung und/oder Synchronisation erreicht werden, dass ein weiter entfernter Anwender B einen ersten, nur unter einem kleinen Betrachtungswinkel einsehbaren Anzeigeinhalt sehen kann, während ein in vorbestimmter Weise vor der Anzeigeeinrichtung stehender Anwender A einen zweiten, unter einem großen Betrachtungswinkel (Weitwinkel) einsehbaren Anzeigeinhalt sehen kann. Eine gegebenenfalls auftretende Überlagerung des ersten und des zweiten Anzeigeinhalts kann mittels geeigneter Gleichtaktung und Synchronisation der Ansteuerung der Anzeigeeinrichtung der Monitoreinrichtung 2 optimiert bzw. unterdrückt werden.

Vorzugsweise kann außerdem das medizintechnische Gerät 1 bzw. die Dialysemaschine eine Erfassungsvorrichtung, etwa eine Kamera, einen Näherungssensor, einen Gewichtssensor, eine Personen- oder Gesichtserkennungseinrichtung, einen Kartenleser oder ein Tastenfeld zur Eingabe einer Kennsequenz oder dergleichen (nicht dargestellt) beinhalten, über welche eine Annäherung eines Anwenders A oder B erfassbar ist. Die Weitwinkeldarstellung der Anzeigeeinrichtung kann sodann vorzugsweise nur bei Erfassung der Anwesenheit des Anwenders A vor dem Gerät aktiviert werden, wobei in diesem Fall auf der Anzeigeeinrichtung dargestellte Information ohne einen sich in ausreichender Nähe befindenden Anwender nur aus der Ferne (und damit nicht im Detail lesbar) sichtbar sein kann, während lesende Naheinblicke unterbunden bleiben.

Wie vorstehend beschrieben wurde, beinhaltet ein medizintechnisches Gerät ein Gehäuse bzw. einen Korpus, im Fall einer Dialysemaschine zur Aufnahme betrieblicher Komponenten für die extrakorporale Blutbehandlung, und eine Monitoreinrichtung mit einer Anzeigeeinrichtung, wobei die Monitoreinrichtung eine Anzeigeinhaltsdarstellungs-Beeinflussungseinrichtung beinhaltet, die dazu angeordnet ist, die Einsehbarkeit von auf der Anzeigeeinrichtung dargestellter Information zu beeinflussen.

Als Vorteile des erfindungsgemäßen Geräts sind unter anderem eine höhere Verfügbarkeit von Informationen, die Darstellung unterschiedlicher Inhalte an einem Monitor, eine Erhöhung des Datenschutzes durch eingeschränkte Sichtbereiche, die Möglichkeit, von jedem Gerät an einem Ort, an dem mehrere Geräte versammelt und/oder vernetzt sind, Parametereinstellungen von einem anderen Gerät aus einzusehen, und die verbesserte Ablesbarkeit von Monitorinformationen auch unter ungünstigen Sicht- und Lichtverhältnissen zu nennen.

## Patentansprüche

1. Vorrichtung zur extrakorporalen Blutbehandlung, insbesondere Dialysemaschine, mit einem Korpus (1) zur Aufnahme betrieblicher Komponenten für die extrakorporale Blutbehandlung, und
einer bezüglich des Korpus (1) nicht ausrichtbaren oder zumindest nicht neigbaren Monitoreinrichtung (2) mit einer Anzeigeeinrichtung (4), wobei
die Monitoreinrichtung (2) eine Anzeigeinhaltsdarstellungs-Beeinflussungseinrichtung (5) aufweist, die dazu angeordnet ist, die Einsehbarkeit von auf der Anzeigeeinrichtung dargestellter Information zu beeinflussen, und die Anzeigeeinrichtung (4) dazu angeordnet ist, zumindest zwei unterschiedliche Anzeigeinhalte gleichzeitig anzuzeigen, **dadurch gekennzeichnet, dass**
die Anzeigeinhaltsdarstellungs-Beeinflussungseinrichtung (5) ein auf einer Anzeigeoberfläche (3) der Anzeigeeinrichtung (4) angeordnetes Entspiegelungsmittel beinhaltet, das dazu angeordnet ist, zumindest einen Teilbereich der Anzeigeoberfläche zu entspiegeln,
die Anzeigeinhaltsdarstellungs-Beeinflussungseinrichtung (5) ferner ein auf der Anzeigeoberfläche (3) der Anzeigeeinrichtung (4) angeordnetes Verspiegelungsmittel beinhaltet, das dazu angeordnet ist, zumindest einen Teilbereich der Anzeigeoberfläche zu verspiegeln, und
die Anzeigeinhaltsdarstellungs-Beeinflussungseinrichtung (5) dazu angeordnet ist, die Sichtbarkeit der auf der Anzeigeeinrichtung (4) gleichzeitig dargestellten zumindest zwei Anzeigeinhalte derart zu beeinflussen, dass
aus einer ersten Blickrichtung und/oder einer ersten Entfernung eines ersten Anwenders (A) auf/zu die/der Anzeigeeinrichtung (4) einer der zumindest zwei Anzeigeinhalte und aus einer zweiten Blickrichtung und/oder einer zweiten Entfernung eines zweiten Anwenders (B) auf/zu die/der Anzeigeeinrichtung (4) ein anderer der zumindest zwei Anzeigeinhalte sichtbar sind, wobei
die Anzeigeinhaltsdarstellungs-Beeinflussungseinrichtung (5) ein zur Änderung seiner Lichttransmissionseigenschaften elektronisch ansteuerbares Filter ist, das als eine Parallaxenbarriere konfiguriert ist.

2. Vorrichtung zur extrakorporalen Blutbehandlung nach Anspruch 1, bei dem das Entspiegelungsmittel (3) eine in dem zumindest einen Teilbereich auf der Anzeigeoberfläche angeordnete Folie oder Beschichtung, oder eine Folie, eine Scheibe, ein Kontraständerungsmittel und/oder eine LED-Anordnung, die vor der Anzeigeoberfläche der Anzeigeeinrichtung angeordnet ist/sind.

3. Vorrichtung zur extrakorporalen Blutbehandlung nach Anspruch 1, bei dem die Anzeigeinhaltsdarstellungs-Beeinflussungseinrichtung (5) ein in die Anzeigeeinrichtung (4) vor oder hinter einem bildgebenden Panel integriertes Lichtrichtungsänderungsfilter, Polarisationsmittel oder Kontraständerungsmittel umfasst, das dazu angeordnet ist, dargestellten Anzeigeinhalt nur aus einer vorbestimmten Position oder Blickrichtung eines Anwenders (A, B) lesbar zu machen.

4. Vorrichtung zur extrakorporalen Blutbehandlung nach einem der vorangehenden Ansprüche, bei dem die Anzeigeeinrichtung (4) als eine Dual-View-Anzeigeeinrichtung konfiguriert ist.

5. Vorrichtung zur extrakorporalen Blutbehandlung nach einem der vorangehenden Ansprüche, bei dem die Anzeigeeinrichtung (4) als eine Split-Screen-Anzeigeeinrichtung konfiguriert ist.

## Claims

1. An apparatus for extracorporeal blood treatment, in particular a dialysis machine, comprising a body (1) for accommodating operational components for the extracorporeal blood treatment, and
wherein a monitor unit (2) which is adapted to be non-orientable or at least to be non-tiltable relative to the body (1) and includes a display unit (4), wherein
the monitor unit (2) includes a display content representation influencing device (5) which is arranged to influence the visibility of information represented on the display unit, and the display unit (4) is arranged to simultaneously display at least two different display contents; **characterized in that**
the display content representation influencing device (5) comprises an antireflection coating means disposed on a display surface (3) of the display unit (4) which is arranged to coat at least a portion of the display surface,
the display content representation influencing device (5) further comprises a silvering means disposed on the display surface (3) of the display unit (4) which is arranged to silver at least a portion of the display surface,
the display content representation influencing device (5) is arranged to influence the visibility of the at least two display contents simultaneously represented on the display unit (4) so that
from a first viewing direction and/or a first distance of a first user (A) to/from the first display unit (4) one of the at least two display contents is visible and from a second viewing direction and/or a second distance of a second user (B) to/from the display unit (4) another one of the at least two display contents is visible, wherein
the display content representation influencing device (5) is a filter adapted to be electronically controlled for changing its light transmission properties, wherein the filter is configured as a parallax barrier.

2. The apparatus for extracorporeal blood treatment according to claim 1, wherein the antireflection coating means (3) is a film or coating disposed in at least a portion of the display surface, or a film, a disk, a contrast changing means and/or an LED array that is/are arranged in front of the display surface of the display unit (4).

3. The apparatus for extracorporeal blood treatment according to claim 1, wherein the display content representation influencing device (5) comprises a light direction changing filter, polarizing means or contrast changing means integrated in the display unit (4) in front of or behind an imaging panel which is arranged to render represented display content readable only from a predetermined position or viewing direction of a user (A, B).

4. The apparatus for extracorporeal blood treatment according to one of the preceding claims, wherein the display unit (4) is configured as a dual-view display unit.

5. The apparatus for extracorporeal blood treatment according to one of the preceding claims, wherein the display unit (4) is configured as a split-screen display unit.

## Revendications

1. Dispositif de traitement extracorporel du sang, en particulier machine de dialyse, avec un corps (1) pour la réception de composants opérationnels pour le traitement extracorporel du sang, et
un dispositif de surveillance (2) non orientable ou au moins non inclinable par rapport au corps (1) avec un dispositif d'affichage (4), dans lequel
le dispositif de surveillance (2) présente un dispositif d'influence de représentation de contenu d'affichage (5) qui est agencé afin d'influencer la visibilité d'informations représentées sur le dispositif d'affichage, et le dispositif d'affichage (4) est agencé afin d'afficher simultanément au moins deux contenus d'affichage différents, **caractérisé en ce que**
le dispositif d'influence de représentation de contenu d'affichage (5) contient un moyen de traitement antireflet agencé sur une surface d'affichage (3) du dispositif d'affichage (4), moyen de traitement antireflet qui est agencé afin de rendre antiréfléchissante au moins une zone partielle de la surface d'affichage,
le dispositif d'influence de représentation de contenu d'affichage (5) contient de plus un moyen d'application de couches réfléchissantes agencé sur une surface d'affichage (3) du dispositif d'affichage (4), moyen d'application de couches réfléchissantes qui est agencé afin d'appliquer une couche réfléchissante à au moins une zone partielle de la surface d'affichage, et
le dispositif d'influence de représentation de contenu d'affichage (5) est agencé afin d'influencer la visibilité des au moins deux contenus d'affichage représentés simultanément sur le dispositif d'affichage (4) de telle manière que
un des au moins deux contenus d'affichage soit visible à partir d'un premier sens d'observation et/ou d'une première distance d'un premier utilisateur (A) sur/par rapport le/au dispositif d'affichage (4) et un autre des au moins deux contenus d'affichage est visible à partir d'un second sens d'observation et/ou d'une seconde distance d'un second utilisateur (B) sur/par rapport le/au dispositif d'affichage (4), dans lequel
le dispositif d'influence de représentation de contenu d'affichage (5) est un filtre commandable électroniquement pour la modification de ses propriétés de transmission de lumière, filtre qui est configuré comme une barrière parallaxe.

2. Dispositif de traitement extracorporel du sang selon la revendication 1, pour lequel le moyen de traitement antireflet (3) est un film ou revêtement agencé dans l'au moins une zone partielle sur la surface d'affichage, ou un film, une vitre, un moyen de modification de contraste et/ou un agencement de DEL qui est/sont agencés devant la surface d'affichage du dispositif d'affichage.

3. Dispositif de traitement extracorporel du sang selon la revendication 1, pour lequel le dispositif d'influence de représentation de contenu d'affichage (5) comprend un filtre de modification de sens de lumière, moyen de polarisation ou moyen de modification de contraste intégré dans le dispositif d'affichage (4) devant ou derrière un panneau de formation d'image qui est agencé afin de rendre lisible un contenu d'affichage représenté seulement à partir d'une position ou sens d'observation prédéterminé d'un utilisateur (A, B).

4. Dispositif de traitement extracorporel du sang selon l'une des revendications précédentes, pour lequel le dispositif d'affichage (4) est configuré comme un dispositif d'affichage à affichage double.

5. Dispositif de traitement extracorporel du sang selon l'une des revendications précédentes, pour lequel le dispositif d'affichage (4) est configuré comme un dispositif d'affichage à écran partagé.
